(19) Europäisches Patentamt
European Patent Office
Office européen des brevets

(11) Veröffentlichungsnummer: **0 381 997**
**A1**

# (12) EUROPÄISCHE PATENTANMELDUNG

(21) Anmeldenummer: **90101390.4**

(51) Int. Cl.⁵: **C12N 9/80**

(22) Anmeldetag: **24.01.90**

(30) Priorität: **04.02.89 DE 3903324**

(43) Veröffentlichungstag der Anmeldung:
**16.08.90 Patentblatt 90/33**

(84) Benannte Vertragsstaaten:
**AT BE CH DE FR GB IT LI NL**

(71) Anmelder: **Degussa Aktiengesellschaft**
**Weissfrauenstrasse 9**
**D-6000 Frankfurt am Main 1(DE)**

(72) Erfinder: **Kula, Maria-Regina, Prof.**
**Selgenbusch 12**
**D-5162 Niederzier(DE)**
Erfinder: **Kittelmann, Matthias, Dr.**
**Kartäuserstrasse 88**
**D-7800 Freiburg(DE)**

## (54) Mikrobiologisch hergestellte N-Acetyl-2,3-didehydroleucin-Acylase, Verfahren zu ihrer Gewinnung und ihre Verwendung.

(57) Gegenstand der Erfindung ist eine mikrobiologisch hergestellte N-Acetyl-2,3-didehydroaminosäure-Acylase und ein Verfahren zu ihrer Gewinnung aus Zoogloea ramigera DSM 4306. Das neue Enzym kann in einem gekoppelten Enzymsystem mit einer L-Leucindehydrogenase zur enzymatischen Umwandlung von N-Acetyl-2,3-didehydroleucin zu L-Leucin über die Zwischenstufen 2-Imino-4-methyl-pentansäure bzw. 2-Keto-4-methyl-pentansäure verwendet werden. Weiterhin kann die Acylase zur Herstellung von D- oder L-Tryptophylglycin aus D- oder L-Tryptophanamid und Glycin sowie von anderen Tryptophyldipeptiden aus Tryptophanamid und einer freien Aminosäure als Aminokomponente eingesetzt werden.

EP 0 381 997 A1

# Mikrobiologisch hergestellte N-Acetyl-2,3-didehydroleucin-Acylase, Verfahren zu ihrer Gewinnung und ihre Verwendung

Die Erfindung betrifft ein bisher nicht beschriebenes Enzym, das Umsetzungen folgender Art katalysiert:

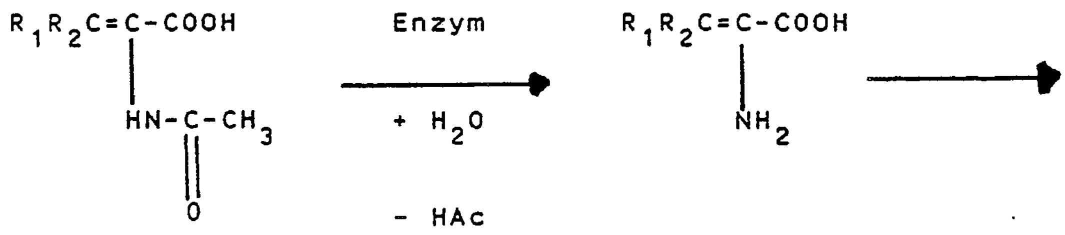


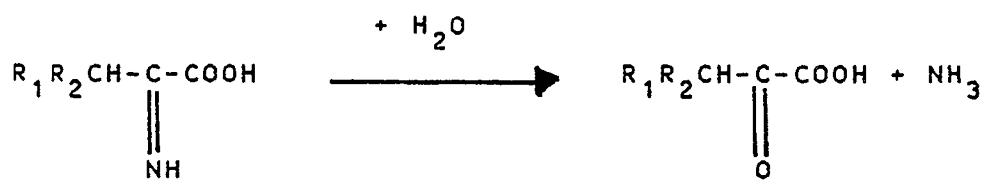


Besonders gute Umsätze erhält man mit $R_1$ = $(CH_3)_2CH$-und $R_2$ = H-, sowie mit $R_1$ = $CH_3$- und $R_2$ = H- (= 2-Acetylaminoacrylsäure).

Nakamichi et al. (Appl. Microbiol. Biotechnol. 19, S. 100 - 105, 1984, und Appl. Biochem. Biotechnol. 11, S. 367 - 376, 1985) sowie Nishida et al. (Enzyme Microb. Technol. 9, S. 479 - 483, 1987) beschreiben das Vorkommen von Acylasen, welche 2-Acetylaminozimtsäure (= N-Acetyl-2,3-didehydropohenylalanin) zu Phenylpyruvat und Essigsäure hydrolysieren, in Bakterienzellen der Species Bacillus sphaericus und Alcaligenes faecalis. Über die Substratspezifität dieser Enzyme wurde nichts veröffentlicht. Hummel et al. (Appl. Microbiol. Biotechnol. 25, S. 175 - 185, 1987) isolierten eine 2-Acetylaminozimtsäure-Acylase aus einem Stamm der Gattung Brevibacterium. Dieses Enzym ist aber nicht in der Lage, andere N-Acetyl-2,3-didehydroaminosäuren zu spalten.

Die erfindungsgemäße N-Acetyl-2,3-didehydroleucin-Acylase ist gekennzeichnet durch die folgenden Eigenschaften:

1) Reaktivität:
sie spaltet die Acetylgruppe von N-Acetyl-2,3-didehydroleucin ab, wobei Essigsäure und nach chemischen Folgereaktionen 2-Keto-4-methyl-pentansäure und Ammoniak als Endprodukte entstehen;

2) Substratspezifität:
sie hydrolysiert sowohl verschiedene N-Acetyl-2,3-didehydroaminosäuren, wie N-Acetyl-2,3-didehydrovalin, -isoleucin, 2-Acetylaminozimtsäure und 2-Acetylaminoacrylsäure als auch Aminosäureamide wie D- und L-Tryptophanamid, D-und L-Leucinamid und L-Methioninamid, nicht aber 2,3-gesättigte N-Acetylaminocarbonsäuren wie N-Acetylleucin oder -valin;

3) Optimaler pH-Wert:
der optimale pH-Wert ist 9,3 ± 1;

4) pH-Stabilität:
sie zeigt gute Stabilität im pH-Bereich zwischen 9 und 10;

5) Optimale Temperatur:
die optimale Temperatur beträgt 55° C bei einem pH-Wert von 9;

6) Temperaturbeständigkeit:
bei 50° sind nach 30 Minuten Inkubation noch 90% Restaktivität nachweisbar;

7) Einflüße von Inhibitoren und Aktivatoren:
inhibierend wirken Hemmstoffe von Serinproteasen, insbesodere Phenylmethansulfonylfluorid (0,001 mM), Glycin beschleunigt die Substratspaltung konzentrationsabhängig;

8) Molekulargewicht:

Das Molekulargewicht beträgt etwa 60000;

9) Untereinheiten:

das Molekul besteht aus nur einer Einheit;

10) $K_M$ -Wert:

der $K_M$-Wert für das Substrat N-Acetyl-2,3-didehydroleucin beträgt 4,5 mM (30° C, 0,1 M Glycinpuffer, pH 9).

Die erfindungsgemäße N-Acetyl-2,3-didehydroleucin-Acylase kann mittels eines Zoogloea-Stammes gewonnen werden, der am 01.12.1987 bei der Deutschen Sammlung von Mikroorganismen in Göttingen unter der Nummer DSM 4306 hinterlegt wurde.

Die folgenden Eigenschaften zeigen, daß der Mikroorganismus der Species Zoogloea ramigera angehört:

Er wächst in leicht gekrümmten, Gram-negativen Stäbchen. Die Zellen sind beweglich durch eine polare Geißel und bilden keine Sporen. Wachstum erfolgt ohne Nitrat strikt aerob. Aus Glucose wird keine Säure gebildet. Katalase- und Oxidasereaktion sowie Nitratreduktion sind positiv, Harnstoffspaltung positiv, Gelatine- und Caseinabbau positiv, Stärkeabbau negativ, Denitrifikation negativ (ungewöhnlich für die Gattung Zoogloea). Der Stamm enthält das Ubichinon Q 9. Der Mikroorganismus kann aufbewahrt werden als lyophilisierte Kultur. Arbeitskulturen werden auf Schrägagar-Röhrchen (N-Acetyl-2,3-didehydroleucin-Medium) gehalten.

Zur Gewinnung der erfindungsgemäßen N-Acetyl-2,3-didehydroleucin-Acylase wird Zoogloea ramigera DSM 4306 in einem wässrigen Nährmedium, das eine Quelle für Kohlenstoff und Stickstoff und Mineralsalze enthält, bei einem Ausgangs-pH-Wert zwischen 7,5 und 9 aerob bei 25 bis 38° C angezogen, dann N-Acetyl-2,3-didehydroleucin als Induktor der Nährlösung zugesetzt und bei pH-Wert 6,5 weiterhin aerob bei 28 bis 38° C kultiviert, die Zellmasse abgetrennt und das Enzym aus den Zellen isoliert.

In größeren Mengen kann das Enzym beispielsweise so gewonnen werden, daß man Zoogloea ramigera in an sich bekannter Weise in einem Bioreaktor gewünschter Größe, z.B. mit 5 l Arbeitsvolumen, anzieht. Für eine erfolgreiche Anzucht sind wichtig:

- eine gute Belüftung (obligat aerober Organismus);
- die Anwesenheit von Nährstoffen, z.B. in komplexer Form als Hefeextrakt;
- ein stufenweises Nachdosieren der Nährstoffe;
- zum Wachstum ein pH-Wert zwischen 7,5 und 9;
- für die Enzymproduktion ein pH-Wert von 6,5;
- für die Enzymproduktion die Anwesenheit von N-Acetyl-2,3-didehydroleucin (5 bis 9 g/l).

Das Enzym kann nach Aufschluß der Zellen durch die Kombination an sich bekannter Methoden der Enzymreinigung gewonnen werden. Das Enzym kann als Bestandteil eines- gekoppelten Enzymsystems mit einer L-Leucindehydrogenase für die enzymatische Umsetzung von N-Acetyl-2,3-didehydroleucin über die Zwischenstufe 2-Imino-4-methyl-pentansäure bzw. die entsprechende Ketosäure zu L-Leucin verwendet werden. Zudem kann das Enzym für die Herstellung von D- oder L-Tryptophylglycin aus D- oder L-Tryptophanamid und Glycin, von D- oder L-Tryptophyl-D-methionin aus D-oder L-Tryptophanamid und D-Methionin sowie von L-Tryptophyl-D-cystein aus L-Tryptophanamid und D-Cystein eingesetzt werden.

Die Erfindung soll durch nachstehende Beispiele näher erläutert werden. Als abgekürzte Formeln werden verwendet N-Acetyldehydro- für N-Acetyl-2,3-didehydro-,ADL für N-Acetyl-2,3-didehydroleucin, ADI für N-Acetyl-2,3-didehydroisoleucin und ADV für N-Acetyl-2,3-didehydrovalin.

## Beispiel 1: Suche nach N-Acetyldehydroleucin-Acylase-Produzenten

63 Boden-, Gewässer- und Klärwerksproben wurden mit einer Mineralsalzlösung aufgeschlemmt bzw. verdünnt, und es wurden mit 0,02 - 0,2 ml dieser Ansätze 20 ml Flüssigmedium (Anreicherungsmedium) beimpft. Die Mineralsalzlösung hatte folgende Zusammensetzung:

| | |
|---|---|
| $K_2HPO_4$ | 3,7 g |
| $KH_2PO_4$ | 1,5 g |
| $MgSo_4 \cdot 7\ H_2O$ | 0,2 g |
| $CaCl_2 \cdot 2\ H_2O$ | 2,0 mg |
| $ZnSo_4 \cdot 7\ H_2O$ | 0,4 mg |
| $FeCl_3 \cdot 6\ H_2O$ | 0,2 mg |
| Deionisiertes Wasser | 1,0 l |
| pH-Wert | 7,2 |

Das Anreicherungsmedium beinhaltete:

| | |
|---|---|
| N-Acetyldehydroaminosäure | 3,0 g |
| Spurenelemente-Lösung | 3,0 ml |
| Hefeextrakt | 0,2 g |
| Mineralsalzlösung (s.o.) | 1,0 l |
| pH-Wert | 7,2 |

Als N-Acetyldehydroaminosäuren wurden das Leucin-, Isoleucin- und Valinderivat eingesetzt. Das Medium wurde vor der Beimpfung in 100 ml Erlenmeyerkolben abgefüllt und durch Autoklavieren sterilisiert. Jedem Kolben wurde nach dem Abkühlen 0,02 ml sterilfiltrierte Vitaminlösung zugesetzt (Zusammensetzung der Vitaminlösung nach Schlegel, H.G. "Allgemeine Mikrobiologie", Thieme Verlag, 1981, S. 169).

Die Kulturen wurden bei 28° C aerob in einem Rundschüttler bei 110 Upm 6 - 10 Tage bebrütet. Dicht bewachsene Ansätze mit einer optischen Dichte bei 660 nm von mindestens 0,7 wurden in sterilem Phosphatpuffer (20 mM, pH 7) in üblicher Weise verdünnt und auf Selektivnährböden mit folgender Zusammensetzung ausplattiert:

| | |
|---|---|
| Agar | 2,0 g |
| Hefeextrakt | 0,1 g |
| Anreicherungsmedium (s.o.) der jeweiligen Kultur | 1,0 l |
| pH-Wert | 7,2 |

Das Medium wurde ohne Vitaminlösung autoklaviert und diese erst nach dem Abkühlen des Agars vor dem Ausgießen in sterile Petrischalen in o.g. Menge zugesetzt. Parallel wurden von jeder Anreicherungskultur wie beschrieben auch Kontrollplatten beimpft, welche die gleiche Zusamensetzung wie die Selektivnährböden aufwiesen, jedoch keine N-Acetyldehydroaminosäuren beinhalteten. Die beimpften Platten wurden 3 bis 10 Tage bei 28° C inkubiert. Von jeder der Kolonientypen, die nur auf den Selektivnährboden, nicht aber auf den Kontrollplatten zu finden waren, wurde eine Kolonie abgenommen und in üblicher Weise auf dem Selektivagar in Reinkultur gebracht (Verdünnungsausstriche, Mikroskopie).

Einheitlich erscheinende Stämme wurden sodann in 100 ml Flüssigmedium (500 ml Erlenmeyerkolben) bei 27° C auf einer Rundschüttelmaschine bei 110 Upm vermehrt. Das Anzuchtmedium hatte folgende Zusammensetzung:

| | |
|---|---|
| Hefeextrakt | 0,5 g |
| Pepton | 0,5 g |
| Anreicherungsmedium (s.o.) des zu testenden Organismus | 1,0 l |
| pH-Wert | 7,2 |

Nach 48 - 60 h wurde der Inhalt der Schüttelkolben zentrifugiert (20 min, 8000 g in einer Kühlzentrifuge)

, und die sedimentierten Zellen wurden in 0,05 M Kaliumphosphatpuffer, pH 7, suspendiert (4 ml Puffer pro 1 g Bakterienfeuchtmasse).

Die Mikroorganismen in dieser Suspension müssen in üblicher Weise aufgeschlossen werden (z.B. Schütteln mit feinen Glasperlen, Ultraschallbehandlung, Frenchpresse). Bei uns wurden die Suspensionen mit Glasperlen (0,1 bis 0,2 mm Durchmesser) versetzt, wobei 2 g Perlen pro 1 ml Zellsuspension verwendet wurden, und dann in einem Reagenzglas für 10 Minuten mit einem Laborschüttler (Typ Reax 2000, Firma Heidolph) gemixt. Mit diesem Verfahren konnten die meisten Organismen gut aufgeschlossen werden. Unlösliche Zellbestandteile und die Glasperlen wurden abzentrifugiert (13000 Upm, Biofuge A, Firma Heraeus) und der Überstand als Enzymquelle (Rohextrakt) genutzt.

Die Ansätze zum Aktivitätstest beinhalteten:

| | |
|---|---|
| 0,2 M N-Acetyldehydroaminosäure | 0,05 ml |
| Rohextrakt | 0,075 ml |
| 0,05 M Tris / HCl-Puffer, pH 7,2 | 0,375 ml |

Alle Rohextrakte wurden zumindest mit N-Acetyldehydroleucin und -isoleucin, meist aber auch mit N-Acetyldehydrovalin getestet. Inkubationszeit und Rohextraktverdünnung wurden so bemessen, daß der Linearitätsbereich des nachgeschalteten Farbtests zum Nachweis des Reaktionsproduktes (0,26 mM Ketosäure) nicht überschritten wurde. Die enzymatische Reaktion wurde in den bei 30° C inkubierten Ansätzen durch Zugabe von 0,25 ml Farbreagenz (1 g/l 2,4-Dinitrophenyhydrazin in 2 N HCl) abgestoppt. Nach weiterer Inkubation bei 30° C für 10 Minuten mit N-Acetyldehydroleucin als Substrat bzw. 25 Minuten mit dem Isoleucin- oder Valinderivat wurden 1,5 ml 2,5 N NaOH zugegeben.

Die durch die enzymatische Hydrolyse freigesetzte Ketosäure bildet mit dem 2,4-Dinitrophenylhydrazin eine im Alkalischen rot gefärbte Schiffsche Base, deren Absorption im Spektralphotometer bei 442 nm gegen einen Ansatz ohne N-Acetyldehydroaminosäure ( = Enzymblank) gemessen wurde. Von diesen Extinktionswerten wurde ein Leerwert abgezogen, den man erhält, wenn man den Extinktionswert eines Ansatzes nur mit Puffer ( = Pufferblank) von dem eines Ansatzes mit Puffer und N-Acetyldehydroaminosäure, aber ohne Rohextrakt ( = Substratblank) abzieht. Anhand einer Eichgeraden mit der entsprechenden Ketosäure wurde die Konzentration des entstandenen Produktes bestimmt. Die Enzymaktivität wird in internationalen Einheiten angegeben, wobei eine Einheit (U) einer Menge von 1 μMol freigesetzter Ketosäure pro Minute entspricht.

Wie Tabelle 1 zeigt, weist der Stamm Zoogloea ramigera ABI 1 (DSM 4306) bei dem oben beschriebenen Testverfahren die höchste Aktivität auf und wurde daher zur Produktion des Enzyms verwendet.

Tabelle 1

Produktion der N-Acetyldehydroaminosäure-Acylase durch die 7 aktivsten Mikroorganismen im Screening

| Substrat: | ADL | | ADI | | ADV | |
|---|---|---|---|---|---|---|
| Stamm | spez. Aktivität (mU/mg) | Volumenausbeute (U/l) | spez. Aktivität (mU/mg) | Volumenausbeute (U/l) | spez. Aktivität (mU/mg) | Volumenausbeute (U/l) |
| ABI 1 | 147 | 11,5 | 19 | 1,41 | 48 | 3,72 |
| ABI 3 | 103 | 11,0 | 15 | 1,62 | 28 | 2,98 |
| ABI 5 | 100 | 9,1 | 8 | 1,21 | 29 | 2,64 |
| SZI 2 | 77 | 4,1 | 13 | 0,70 | 27 | 1,47 |
| SZI 4 | 109 | 7,1 | 12 | 0,81 | 33 | 2,14 |
| SZI 5 | 69 | 4,2 | 15 | 0,90 | 35 | 2,12 |
| SZI 7 | 129 | 10,9 | 15 | 1,49 | 30 | 2,94 |

Beispiel 2: Produktion der N-Acetyldehydroleucin-Acylase

a) Acylasefermentation mit Stamm ABI 1 im 5 l Maßstab

Verwendet wurde ein Bioreaktor mit 5 l Arbeitsvolumen, der mit einer Vorrichtung zur automatischen pH-Wert-Regulierung und Schaumbekämpfung ausgestattet war. Das Medium enthielt:

| | |
|---|---|
| Hefeextrakt | 35,0 g |
| Mineralsalzlösung (siehe Beispiel 1) | 5,0 l |
| pH-Wert | 8,3 |

Nach Sterilisation wurde das Medium mit 500 ml einer Vorkultur angeimpft, die 60 Stunden in Portionen zu 150 ml des gleichen Mediums in 500 ml Erlenmeyerkolben angezogen worden war. Die Inkubation war bei 30° C und 110 Upm auf einem Rundschüttler erfolgt.

Die Bedingungen während der Wachstumsphase der Fermentation waren:

| | |
|---|---|
| pH-Wert | 8,3 |
| Temperatur: | 35,0° C |
| Konzentration des gelösten Sauerstoffs (% Sättigung) | 80 % |
| maximale Rührerdrehzahl | 300 Upm |

Zu verschiedenen Zeiten wurden Proben genommen und das Zellwachstum durch Trübungsmessung (Messung der optischen Dichte) bei 660 nm verfolgt.

Nach 10, 21 und 24 Stunden wurden jeweils 300 ml einer konzentrierten, durch Autoklavieren sterilisierten Hefeextraktlösung (100 g/l in Mineralsalzlösung, wie sie in Beispiel 1 beschrieben ist) dem Fermenterinhalt unter keimfreien Bedingungen zudosiert.

Nach etwa 25 bis 30 Stunden Bebrütung, als keine weitere Zunahme der Bakterienmasse mehr zu verzeichnen war, wurden 3,5 l des Mediums mittels einer steril gekoppelten Querstrom-Mikrofiltrationsanlage (Typ Sartocon II, 0,6 qm Polyolefinmembran, Porendurchmesser 0,2 um, Firma Sartorius) abfiltriert. Hierbei wurde der Fermenterinhalt mit einer Schlauchpumpe mit 185 1/h durch das Filtrationsmodul gefördert, so daß eine Druckdifferenz von 0,8 - 0,9 Bar zwischen Moduleingang und -ausgang hervorgerufen wurde. Die Bakterienzellen und das nicht abgetrennte Flüssigmedium wurden in den Fermenter zurückgeführt. Zuvor waren die Schlauchverbindungen zwischen Fermenter und Filtrationseinheit einschließlich des Pumpenschlauches durch Autoklavieren und die Filtrationsanlage mit hindurchströmendem Wasserdampf (1,2 Bar Überdruck, 30 Minuten) sterilisiert worden.

Die aufkonzentrierte Biomasse im Reaktor wurde durch Diafiltration mit 2 Liter steriler Mineralsalzlösung (siehe Beispiel 1) gewaschen und dann der Fermenter mit Induktionsmedium aufgefüllt. Das Induktionsmedium war wie folgt zusammengesetzt:

| | |
|---|---|
| N-Acetyldehydroleucin | 25 g/l |
| Hefeextrakt | 35 g/l |
| Mineralsalzlösung | 4,5 l |
| pH-Wert | 6,5 |

Die Bedingungen zur Enzyminduktion waren:

| | |
|---|---|
| pH-Wert | 6,5 |
| Temperatur | 31,5° C |
| Konzentration des gelösten Sauerstoffs (% Sättigung) | 80 % |
| maximale Rührerdrehzahl | 300 Upm |

Zu verschiedenen Zeiten wurden Proben genommen und nach Trübungsmessung (optische Dichte bei 660 nM) und einem Test auf Acylaseaktivität der maximal erreichbare Enzymgehalt und der beste Erntezeitpunkt bestimmt. Die Acylase-Tests wurden mit 0,02 M N-Acetyldehydroleucin in 0,1 M Glycin/Natriumhydroxydpuffer, pH 10, angesetzt und, wie unter Beispiel 1 beschrieben, durchgeführt.

Es zeigte sich, daß die Acylase nur während der 2. Fermentationsphase gebildet wird und die Enzymaktivität etwa 24 Stunden nach Zugabe des Induktionsmediums den maximalen Wert erreicht.

b) Gewinnung des Rohextraktes

Die Bakterien-Feuchtmasse (133 g) wurde in 50 mM Glycinpuffer, pH 11, suspendiert, so daß die Konzentration der Zellsuspension 40 %ig war (Endvolumen 333 ml). Die Zellinhaltsstoffe wurden in der gekühlten Suspension (ca. 4° C) durch einen mechanischen Zellaufschluß in einer Glasperlenmühle der Firma Bachofen (Dyno-Mill, Typ KLD) freigesetzt. Hierbei wurde der 680 ml fassende Mahlbehälter mit 0,3 mm großen Glasperlen gefüllt, so daß sich ein Schüttvolumen von 578 ml ergab (85 %). Der Aufschluß wurde bei einer Rührerwellendrehzahl von 3000 UpM durchgeführt, wobei der Kühlmantel des Mahlbehälters sowie das Rührwellenlager während des Laufens mit Ethylenglykollösung von -20° C gekühlt wurden, um eine Erwärmung des Produktes weitgehend zu vermeiden. Nach 4 Minuten Aufschlußzeit war ein Desintegrationsgrad von über 90 % erreicht. Die Glasperlen wurden durch 2 Minuten Zentrifugation bei 3000 g abgetrennt und zweimal gewaschen, indem sie jeweils mit 192 ml Glycinpuffer vermischt und erneut abzentrifugiert wurden. Die Überstände der Zentrifugationsschritte wurden vereinigt und ein Großteil der Zelltrümmer durch 30 Minuten Zentrifugation bei 12000 g in einer Kühlzentrifuge abgetrennt. Der zu 50 % (w/v) mit Glycerin versetzte Rohextrakt konnte bei -20° C über Monate ohne Aktivitätsverlust gelagert werden.

Beispiel 3: Wachstum von Zoogloea ramigera ABI 1

a) Wachstum bei verschiedenen Start-pH-Werten

In einem Medium aus Mineralsalzlösung (s. Beispiel 1) und 7 g/l Hefeextrakt wurde der pH-Wert von 6,5 - 10 in Schritten zu 0,5 Einheiten variiert. Von den auf der Rundschüttelmaschine mit 110 UpM bei 30° C bebrüteten Kulturen (30 ml in 100 ml Erlenmeyerkolben) wurden zu verschiedenen Zeiten Proben entnommen, in denen die Bakterienmasse durch Messung der optischen Dichte bei 660 nm bestimmt wurde.

Nach 43 h Wachstum wurde im pH-Wert-Bereich zwischen 8 und 8,5 die höchste Zelldichte erreicht.

b) Wachstum bei verschiedenen Hefeextraktkonzentrationen

Das Wachstum von Stamm ABI 1 wurde in Schüttelkulturen (wie unter a) beschrieben) mit einem Medium aus Mineralsalzlösung (siehe Beispiel 1) und 7 - 25 g/l Hefeextrakt mit einem Start-pH-Wert von 8,25 durch Messung der optischen Dichte bei 660 nm verfolgt.

Es zeigte sich, daß bei Hefeextraktkonzentrationen von 15 g/l und mehr der Mikroorganismus verzögert anwächst.

Beispiel 4: Induktion der N-Acetyldehydroleucin-Acylase

a) Veränderung des Induktors

In Schüttelkulturen mit einem Medium aus Mineralsalzlösung (siehe Beispiel 1) mit 0,5 g/l Hefeextrakt und 0,5 g/l Pepton bei pH-Wert 7 wurden verschiedene Substanzen in einer Konzentration von 3 g/l als Induktoren eingesetzt. Die Schüttelkulturtechnik ist in Beispiel 1 a) beschrieben.

Wie Tabelle 2 zeigt, weist N-Acetyldehydroleucin das höchste Induktionsvermögen auf. N-Acetylaminosäuren oder 2-Acetylaminozimtsäure sind nur schwach wirksam.

Tabelle 2

| Acylase-Aktivität nach 60 Stunden Bebrütung in Abhängigkeit vom Induktor | | | |
|---|---|---|---|
| Induktor | U/mg | Induktor | U/mg |
| Ad-leucin | 0,222 | Ac-L-leucin | 0,018 |
| AD-isoleucin | 0,172 | Ac-D,L-tryptophan | 0,018 |
| AD-valin | 0,121 | 2-Acetylaminozimtsäure | 0,016 |
| Ac-L-isoleucin | 0,039 | Ac-D,L-tyrosin | 0,011 |
| Ac-D,L-valin | 0,031 | ohne | 0,015 |
| Ac-D,L-phenylalanin | 0,031 | | |
| AD: N-Acetyldehydro-; Ac-: N-Acetyl-: | | | |

b) Änderung des Start-pH-Wertes

Im Mineralsalzmedium mit 3 g/l N-Acetyldehydroleucin und 2 g/l Hefeextrakt wurde der pH-Wert von 5,5 - 9 in Schritten zu 0,5 Einheiten verändert.

Die Enzymproduktion erreicht bei pH 6,5 ihr Optimum und sinkt mit zunehmendem pH-Wert steil ab, wie in Tabelle 3 dargestellt ist.

Tabelle 3

| Abhängigkeit der Acylasebildung durch den Stamm ABI 1 vom pH-Wert | | |
|---|---|---|
| pH-Wert | U/mg | U/l |
| 5,5 | n.b. | n.b. |
| 6,0 | n.b. | n.b. |
| 6,5 | 0,202 | 14,6 |
| 7,0 | 0,136 | 13,3 |
| 7,5 | 0,115 | 10,8 |
| 8,0 | 0,075 | 7,6 |
| 8,5 | 0,069 | 5,3 |
| 9,0 | 0,064 | 5,3 |
| n.b.: nicht bestimmt, da zu geringes Wachstum | | |

c) Änderung der N-Acetyldehydroleucin-Konzentration

Zoogloea ABI 1 wurde im Mineralsalzmedium mit Start-pH-Wert 8 und 7 g/l Hefeextrakt bei 30° C mit der Rundschüttelmaschine angezogen. Nach 48 Stunden wurden die Zellen 10 Minuten bei 8000 g abzentrifugiert, im o.g. Medium mit verschiedenen Konzentrationen an N-Acetyldehydroleucin und einem pH-Wert von 6,5 resupendiert und für weitere 16 Stunden im Rundschüttler bebrütet.

Tabelle 4 zeigt, daß ab 5 g/l N-Acetyldehydroleucin keine weitere Zunahme der Enzymproduktion zu verzeichnen ist.

Tabelle 4

| Acylaseaktivität in Abhängigkeit von der N-Acetyldehydroleucin-Konzentration in zweistufiger Schüttelkultur | | |
|---|---|---|
| N-Acetyldehydroleucin (g/l) | Acylaseproduktion | |
| | U/mg | U/l |
| ohne | 0,015 | 5 |
| 3 | 0,140 | 41 |
| 5 | 0,189 | 70 |
| 7 | 0,190 | 65 |
| 9 | 0,204 | 72 |

Beispiel 5: Aufreinigung der Acylase

a) Fällung der Nukleinsäuren mit Polyethylenimin

Der durch Fermentation, Zellaufschluß in der Glasperlenmühle und Zentrifugation gewonnene Rohextrakt (666 ml) wurde im Eisbad gekühlt und mit 35 ml einer 10 %igen Polyethyleniminlösung (rel. Molekülmasse 30 - 40 • $10^3$) mit einem pH-Wert von 11 versetzt, 5 Minuten bei 0° C inkubiert und die ausgefallenen Nukleinsäuren sowie bisher noch nicht abgetrennte Zelltrümmer durch 30 Minuten Zentrifugation in der Kühlzentrifuge bei 18000 g sedimentiert.

Durch die Nukleinsäurefällung konnten bei der nachfolgenden Ammoniumsulfatfraktionierung die Ausbeute um 18,6 % und der Anreicherungsfaktor um 206 % gesteigert werden.

b) Proteinfällung mit Ammoniumsulfat

Der Überstand (690 ml) wurde mit 460 ml einer gesättigten Ammoniumsulfatlösung (761 g/l), deren pH-Wert durch Zugabe von festem Natriumhydroxyd auf 9 eingestellt worden war, versetzt und 30 Minuten im Eisbad gerührt. Das ausgefallene Protein wurde 30 Minuten bei 15000 g in der Kühlzentrifuge sedimentiert und in 200 ml 50 mM Glycinpuffer mit pH-Wert 11 gelöst.

c) Aussalzchromatographie auf Sepharose CL-4B

Dem konzentriert gelösten Proteinpräzipitat wurde solange gesättigte Ammoniumsulfatlösung zugesetzt, bis eine Leitfähigkeit von 80 - 90 mS/cm erreicht war, was bei pH-Wert 11 einer Ammoniumsulfatkonzentration von etwa 25 % Sättigung entspricht. Ausgefallenes Protein wurde bei 15000 g (30 Minuten) abzentrifugiert. 125 ml des Überstandes (250 ml insgesamt) wurden auf eine Sepharose CL-4B-Säule (2,6 x 22,6 cm) aufgetragen, die mit 25 % Ammoniumsulfat (pH-Wert 11) äquilibriert war. Die Elution erfolgte durch Anlegen eines von 26 auf 0 % Ammoniumsulfat absteigenden Gradienten (500 ml). Das Eluat wurde in Fraktionen zu 5 ml aufgefangen. Die Acylase desorbierte bei 17 - 19 % Ammoniumsulfat-Sättigung vom Chromatographie-Gel. Die aktiven Fraktionen (25 ml) wurden vereinigt und mit gesättigter Ammoniumsulfatlösung (pH 9) auf das doppelte Volumen verdünnt. Nach 30 Minuten Inkubation im Eisbad wurde das ausgefällte Protein abzentrifugiert und in 2 ml 50 mM Glycinpuffer mit pH 11 aufgenommen. Das konzentrierte Acylasepräparat wurde zu 43,5 % (w/v) mit Glycerin versetzt und bei -20° C gelagert.

Da beim hier beschriebenen Chromatographieverfahren nur 10 - 17 % des aufgetragenen Proteins an die Säule binden, ist es möglich, mit einer relativ kleinen Säule große Proteinmengen aufzuarbeiten.

d) Analytische Fast Protein Liquid Chromatography (FPLC) an Mono-Q

0,255 ml des konzentrierten und mit Glycerin versetzten Acylasepräparates wurden mit 1,13 % (v/v) Triton X-100 in 50 mM Glycinpuffer (pH 11) auf 2 ml verdünnt und auf die Mono-Q-Säule (1 ml) aufgegeben, welche mit o.g. Glycinpuffer, ergänzt mit 0,2 % Triton X-100 , äquilibriert war. Eluiert wurde mit einem von 0 auf 0,15 M ansteigenden $Na_2SO_4$-Gradienten (40 ml), wobei die Acylase bei 0,047 - 0,050 M $Na_2SO_4$ von der Säule gewaschen wurde. Nach Vereinigung der aktiven Fraktionen wurde das gereinigte Enzym teilweise zur Lagerung bei -20° C mit 43,5 % (w/v) Glycerin versetzt und teilweise zur Aufbewahrung bei 4-8° C im Kühlschrank zu 25 % (w/v) glyceriniert. Unter letzteren Bedingungen war in 76 Tagen kein Aktivitätsverlust zu verzeichnen.

Tabelle 5 zeigt die Ergebnisse der Aufreinigung.

Tabelle 5

Aufreinigungsschema für die N-Acetyldehydroleucin-Acylase

| Probe/Reinigungsschritt | Gesamtenzym ( U ) | Ausbeute ( % ) | spez.Aktivität ( U/mg ) | Anreicherungsfaktor |
|---|---|---|---|---|
| Rohextrakt | 1963 | 100 | 0,348 | 1 |
| Nukleinsäurefällung (0,9% PEI) | 1810 | 92 | 0,376 | 1,08 |
| Ammoniumsulfatfällung (0-40%) | 1505 | 77 | 1,27 | 3,65 |
| Aussalzchromatogr. (26-0% Ammoniumsulfat) | 785 | 40 | 12,0 | 34,5 |
| Konzentrierung (Fällung mit 60% Ammoniumsulfat) | 785 | 40 | 13,3 | 38,2 |
| FPLC-Mono-Q (0-0,15 M Natriumsulfat in 0,2 % Triton X-100) | 557 | 28 | 74-109 | 213-313 |

PEI: Polyethylenimin, rel. Molekülmasse: 30 - 40 • $10^3$

Beispiel 6: Abhängigkeit der Reaktionsgeschwindigkeit vom pH-Wert

Die Reaktionsgeschwindigkeit der hydrolytischen Abspaltung von Essigsäure aus der Verbindung N-Acetyldehydroleucin in Gegenwart der ADL-Acylase wurde in Abhängigkeit vom pH-Wert im Reaktionsgemisch bestimmt. Der Testansatz war wie folgt zusammengesetzt:

| | | |
|---|---|---|
| 200 mM | N-Acetyldehydroleucin in 20 mM Tris/Phosphorsäurepuffer (pH 9) | 0,05 ml |
| 1,0 U/ml | Acylase | 0,01 ml |
| 0,1 M | Puffer | 0,44 ml |

Vor dem Zusammenmischen der Reaktionsansätze wurden verschiedene pH-Werte im Bereich von 5 bis 7,5 in Kaliumphosphatpuffer, von 7,5 bis 9 in Trispuffer und von 7 bis 12 in Glycinpuffer durch Zugabe von Natriumhydroxid bzw. Phosphorsäure eingestellt. Die unter Testbedingungen vorliegenden pH-Werte wurden in Kontrollansätzen ohne Enzym gemessen. Nach 10 Minuten Reaktionszeit bei 30° C wurden die Enzymaktivitäten durch colorimetrische Messung der Ketosäurekonzentration (siehe Beispiel 1) bestimmt.

Das Optimum der Reaktionsgeschwindigkeit im Glycinpuffer liegt im pH-Bereich zwischen 8,6 und 10,1, in den glycinfreien Puffern zwischen 7,7 und 9,1.

Beispiel 7: Optimale Reaktionstemperatur

Testansätze mit 20 mM N-Acetyldehydroleucin in 0,1 M Glycinpuffer (pH 9) wurden 5 Minuten bei Temperaturen zwischen 10 und 70° C vortemperiert und dann die Enzymreaktion durch Acylasezugabe gestartet. Bei Temperaturen von 10 bis 40° C enthielten die Ansätze 0,0666 U/ml Enzym, bei 40 bis 70° C 0,02 U/ml. Nach 2 Minuten wurden die Enzymreaktionen durch Zugabe von eisgekühltem Nachweisreagenz gestoppt, die Teströhrchen im Eisbad heruntergekühlt und nach 15 Minuten Inkubation bei 30° C wie gewöhnlich zur Farbentwicklung alkalisiert (vgl. Beispiel 1).

Die maximale Reaktionsgeschwindigkeit wird bei 55° C erreicht und ist um den Faktor 3,1 höher als bei der Standardtemperatur von 30° C.

Beispiel 8: Stabilität der N-Acetyldehydroleucin-Aoylase

a) pH-Stabilität

Die pH-Stabilität der ADL-Acylase wurde im pH-Bereich von 7 bis 12 untersucht. An Mono-Q gereinigtes Enzym wurde mit verschiedenen Puffern unterschiedlicher pH-Werte 20-fach verdünnt und 3 Wochen bei 25° C gelagert. Zu verschiedenen Zeiten wurden Proben entnommen und deren Enzymaktivität unter Standardbedingungen getestet. Für die unterschiedlichen pH-Werte wurden folgende Puffer verwendet:

| | | |
|---|---|---|
| 0,1 M | Kaliumphosphat | pH 7,0, 7,5 |
| 0,1 M | Tris/Phosphorsäure | pH 7,5, 8,0, 8,5, 9,0 |
| 0,1 M | Glycin | pH 9,0, 9,5, 10,0, 10,5, 11,0, 11,5, 12,0 |

Das Zusammenmischen der Ansätze, deren Lagerung und die Probenahme erfolgten unter sterilen Bedingungen. Die Standardbedingungen zum Enzymtest waren:
20 mM N-Acetyldehydroleucin
Acylase in limitierender Menge, so daß der Linearitätsbereich des nachfolgenden Farbtests (siehe Beispiel 1) nicht überschritten wurde
0,1 M Glycinpuffer

pH-Wert 9

Wie in Tabelle 6 ersichtlich ist, konnten im pH-Bereich von 9 - 10,5 nach 3 Wochen noch ca. 30 - 40 % der ursprünglichen Acylaseaktivität nachgewiesen werden.

Tabelle 6

| pH-Stabilität der N-Acetyldehydroleucin-Acylase | | | | | |
|---|---|---|---|---|---|
| Puffer pH-Wert | Restaktivität (%) nach | | | | |
| | 1 Tag | 3 Tagen | 1 Woche | 2 Wochen | 3 Wochen |
| Kaliumphosphat (0,1 M) | | | | | |
| 7,0 | 78 | 16 | 0 | 0 | 0 |
| 7,5 | 83 | 39 | 0 | 0 | 0 |
| Tris/Phosphorsäure (0,1 M) | | | | | |
| 7,5 | 61 | 37 | 0 | 0 | 0 |
| 8,0 | 60 | 44 | 7 | 0 | 0 |
| 8,5 | 63 | 62 | 37 | 13 | 9 |
| 9,0 | 92 | 80 | 56 | 25 | 27 |
| Glycin (0,1 M) | | | | | |
| 9,0 | 97 | 91 | 64 | 35 | 30 |
| 9,5 | 89 | 103 | 68 | 35 | 37 |
| 10,0 | 102 | 99 | 68 | 34 | 43 |
| 10,5 | 98 | 95 | 57 | 28 | 25 |
| 11,0 | 86 | 86 | 54 | 15 | 7 |
| 11,5 | 71 | 69 | 35 | 5 | 2 |
| 12,0 | 4 | 3 | 0 | 0 | 0 |

b) Temperaturbeständigkeit der Acylase

Die Acylase wurde 30 Minuten bei Temperaturen von 10 bis 70° C inkubiert, und anschließend wurde der Aktivitätstest unter Standardbedingungen (siehe Beispiel 8a)) durchgeführt.

Nach 30 Minuten bei 50° C sind noch 89 % der Ausgangsaktivität nachzuweisen, bei höheren Temperaturen wird die Acylase rasch desaktiviert.

c) Stabilität der Acylase in Anwesenheit verschiedener Anionen

Der Einfluß verschiedener Anionen auf die Stabilität der ADL-Acylase wurde untersucht. Rohextrakt, der zu 43,5 % (w/v) mit Glycerin versetzt worden war, wurde 1:10 mit 0,5 molaren Lösungen verschiedener Natriumsalze in 50 mM Glycin (pH 11) verdünnt und bei Raumtemperatur (20 bis 25° C) gelagert. Nach verschiedenen Zeiten wurden Proben entnommen und deren enzymatische Aktivität unter Standardbedingungen, wie in Beispiel 8a) beschrieben, gemessen.

Es zeigte sich, daß die Stabilität der Acylase mit der Ladung und Größe des Anions zunimmt (siehe Tabelle 7).

Tabelle 7

| Stabilität der N-Acetyldehydroleucin-Acylase in Anwesenheit verschiedener Natriumsalze | | | |
|---|---|---|---|
| Natriumsalz (0,5 M) | % Restaktivität nach | | |
| | 3 Tagen | 6 Tagen | 21 Tagen |
| Phosphat | 96 | 105 | 27 |
| Sulfat | 89 | 94 | 15 |
| Acetat | 86 | 81 | 6 |
| Formiat | 76 | 63 | 2 |
| Chlorid | 35 | 0 | 0 |

Beispiel 11: Einflüsse von Inhibitoren und Aktivatoren

a) Einfluß von Chelatbildnern, Metallkationen und Enzymhemmstoffen

Der Einfluß verschiedener Zusatzstoffe auf die Reaktionsgeschwindigkeit der Spaltung von N-Acetyldehydroleucin wurde unter Standardbedingungen, wie im Beispiel 8 a) beschrieben, gemessen.

Aus Tabelle 8 geht hervor, daß lediglich Inhibitoren von Serinhydrolasen die Acylase stark hemmen, insbesondere Phenylmethansulfonylfluorid noch in mikromolarer Konzentration.

Tabelle 8: Einfluß von Zusatzstoffen auf die N-Acetyldehydroleucin-Acylaseaktivität

| Hemmstoff | % Restaktivität | | |
|---|---|---|---|
| | 1 mM | 10 mM | 100 mM |
| **Komplexbildner:** | | | |
| EDTA | n.b. | 95 | n.b. |
| Citrat | 99 | 84 | n.b. |
| Bipyridin | 91 | 70 | n.b. |
| Phenanthrolin | 94 | 52 | n.b. |
| $NaN_3$ | 105 | 103 | n.b. |
| **Bivalente Kationen:** | | | |
| $CaCl_2$ | n.b. | 93 | n.b. |
| $CuSO_4$ | 87 | 83 | n.b. |
| $CoCl_2$ | 91 | 85 | n.b. |
| $MgCl_2$ | 95 | 88 | n.b. |
| $MnCl_2$ | 99 | 87 | n.b. |
| $ZnCl_2$ | 95 | 82 | n.b. |
| **reduzierende Agenzien:** | | | |
| MeSH | 93 | 99 | 81 |
| Glutathion reduziert | 95 | 95 | n.b. |
| Dithiothreitol | 103 | 58 | n.b. |
| **SH-Gruppenreagenzien:** | | | |
| pCMB | 104 | 98 | n.b. |
| pOHMB | 93 | 91 | n.b. |
| Jodacetamid | 104 | 74 | n.b. |
| Jodacetat | 76 | 49 | n.b. |
| $HgCl_2$ | 84 | 70 | n.b. |
| KCN | 105 | n.b. | n.b. |

Tabelle 8 (Fortsetzung):

| Hemmstoff | % Restaktivität | | |
|---|---|---|---|
| | 1 mM | 10 mM | 100 mM |
| **Inhibitoren von PLP-Enzymen:** | | | |
| Cycloserin | 111 | 105 | n.b. |
| Semicarbazid | 113 | 93 | n.b. |
| **Inhibitoren von Serin-Hydrolasen:** | | | |
| Neostigmin | 102 | 82 | 0 |
| pABA*) | n.b. | 89 | 54 |
| PMSF | 0,0001 mM: | 0,01 mM: | |
| | 10 | 0 | n.b. |

*) pABA zu 200 mM: 0 % Restaktivität

MeSH: Mercaptoethanol,
pABA: para-Aminobenzamidin,
pCMB: para-Chloromercuribenzoat,
pOHMB: para-Hydroxymercuribenzoat,
PLP: Pyridoxalphosphat,
PMSF: Phenylmethansulfonylfluorid

b) Einfluß von Glycin auf die Reaktionsgeschwindigkeit

Der Einfluß von Glycin in 0 bis 1,2 molarer Konzentration, gelöst in 0,1 M Tris/HCl-Puffer, wurde unter Standardbedingungen (Beispiel 8a) ermittelt.

Glycin beschleunigt die Spaltung von N-Acetyldehydroleucin durch die ADL-Acylase maximal 3,3 fach. Die Glycinkonzentration, welche für halbmaximale Reaktionsbeschleunigung erforderlich ist, beträgt 90 mM (Berechnung nach Ingami, T. und Murachi, T., J. Biol. Chem. 238 (5), 1905 - 1907, 1963).

Beispiel 12: Bestimmung des Molekulargewichts und der Anzahl an Untereinheiten

Das Molekulargewicht des nativen Enzyms wurde durch Gelfiltration an Sephacryl S-200 HR ermittelt. Die an ein FPLC-System gekoppelte Säule (1,6 x 69,6 cm) wurde mit einer Durchflußrate von 1 ml/Minute betrieben, wobei 0,1 ml des mittels Aussalzchromatographie gereinigten und glycerinierten Enzyms nach 2-facher Verdünnung als Probe dienten. Als Eichproteine dienten Cytochrom C (Pferd), Myoglobin (Wal), Myoglobin (Pferd), Aldolase (Kaninchenmuskel), Carboanhydrase und Rinderserumalbumin. Das Molekulargewicht der Acylase beträgt 65000 ± 5000.

In der Gelelektrophorese in Gegenwart von Natriumdodecylsulfat (SDS) wurde für das denaturierte Enzym ein Molekulargewicht von 55000 ± 4000 ermittelt. Demnach besteht die Acylase aus einer Polypeptidkette von ungewähnlicher Länge. Für die Eichkurve wurde $\alpha_2$-Makroglobulin (Pferdeplasma), Phosphorylase b (Kaninchenmuskel), Glutamat-Dehydrogenase (Rinderleber), Lactatdehydrogenase (Schweinemuskel) und Trypsininhibitor (Sojabohne) verwendet.

Beispiel 13: Substratspezifität der N-Acetyldehydroleucin-Acylase

a) Abhängigkeit der Acylaseaktivität von der Konzentration verschiedener N-Acetyldehydroaminosäuren

Die Aktivität der Acylase wurde unter Standardbedingungen (siehe Beispiel 8 a)) mit verschiedenen N-Acetyldehydroaminosäuren in Konzentrationen von 0,1 bis 300 mM bestimmt. Eine zweite Meßreihe mit N-Acetyldehydroleucin im o.g. Konzentrationsbereich wurde in 0,1 M Tris/Phosphorsäure-Puffer angesetzt.

Der $K_M$-Wert für ADL beträgt in Glycinpuffer 4,5 mM. In Tabelle 9 sind die kinetischen Daten für die Spaltung der Acetyldehydroaminosäuren zusammengestellt.

Tabelle 9

| Kinetische Daten der N-Acetyldehydroleucin-Acylase | | | | |
|---|---|---|---|---|
| Substrat | Puffer | % $V_{max.}$ | $K_M$ (mM) | $K_I$ (mM) |
| ADL | Glycin, 0,1 M | 100 | 4,53 | 418 |
| ADI | Glycin, 0,1 M | 14 | 5,77 | 140 |
| ADV | Glycin, 0,1 M | 23 | 20,9 | 732 |
| ACA | Glycin, 0,1 M | 46 | 2,62 | 942 |
| ADA | Glycin, 0,1 M | 90 | 6,68 | 714 |
| ADL | Tris-Phosphorsäure, 0,1 M | 62 | 7,01 | 2564 |

Legende siehe folgende Seite

Reaktionsbedingungen: 0,1 M Glycin, pH 9, 30° C. Anpassung an die Gleichung $v = V_{max.} \cdot S/(K_M + S + S^2/K_I)$ (Cleland, W., 1963, Methods in Enzymology 63, 103 -138).

ADL: Acetyldehydroleucin,
ADI: Acetyldehydroisoleucin,
ADV: Acetyldehdydrovalin,
ACA: Aoetylaminozimtsäure,
ADA: Acetyldehydroalanin ( = N-Acetylaminoacrylsäure).

b) Hydrolyse anderer Verbindungen durch die N-Acetyldehydroleucin-Acylase

Für 81 Verbindungen wurde qualitativ überprüft, ob sie als Substrat von der Acylase akzeptiert werden. Die Testansätze beinhalteten:

| | |
|---|---|
| 50 mM | Testsubstrat |
| 0,343 U/ml | Acylase |
| 0,1 M | Tris/HCl-Puffer |
| pH 9 | |

Nach 16 und 40 Stunden Inkubation bei 25° C wurden den Ansätzen Proben entnommen, von denen

die meisten dünnschichtchromatographisch (70 % (v/v) Ethanol als Laufmittel) im Vergleich mit Substratstandards analysiert wurden. Die Detektion erfolgte mit Ninhydrin-Sprühreagenz. Einige Proben wurden zusätzlich oder alternativ mit dem Aminosäureanalysator untersucht.

Die Testansätze zur Hydrolyse von Hydantoinen hatten folgende Zusammensetzung:

| | |
|---|---|
| 25 mM | Substrat oder Puffer |
| 1 U/ml | Acylase |
| 50 mM | Tris-Phosphorsäurepuffer |
| pH 9 | |
| Gesamtvolumen 100 µl | |

Als Substrate wurden Isopropylhydantoin, Hydantoinsäure und Dihydrouracil eingesetzt. Nach 11,5 Stunden bei 30° C wurden 175 µl 12 % (w/v) Trichloressigsäure und 25 µl Nachweisreagenz (10 % (w/v) p-Aminobenzaldehyd in 6 N HCl) zugesetzt, ausgefallenes Protein in einer Eppendorf-Tischzentrifuge abzentrifugiert und in den Überständen die Absorption bei 450 nm gemessen.

Zur Bestimmung der enzymatischen Hydrolyserate von 4-Nitrophenylacetat wurde die Geschwindigkeit der Extinktionszunahme (402 nm) durch das freigesetzte 4-Nitrophenol im nachstehenden Ansatz gemessen:

| | |
|---|---|
| 20 mM | 4-Nitrophenyacetat |
| 0,2 U/ml | Acylase |
| 0,1 M | Kaliumphosphatpuffer |
| pH-Wert 7 | |
| 25° C | |

Um die chemische Hydrolyse zu berücksichtigen, wurde vom so ermittelten Wert die Extinktionszunahmerate in einem Ansatz ohne Acylase abgezogen. Anhand einer Eichgeraden mit 4-Nitrophenylacetat wurde die Enzymaktivität berechnet.

Die aus den Aminosäureamiden durch Acylaseeinwirkung entstandenen Aminosäuren wurden mit dem Aminosäureanalysator quantitativ erfaßt.

Für die Bestimmung der relativen Aktivitäten wurde die Hydrolyserate mit N-Acetyldehydroleucin unter vergleichbaren Reaktionsbedingungen gleich 100% gesetzt.

Lediglich Tryptophanamid wird mit einer Reaktionsrate in der Größenordnung gespalten, wie sie mit N-Acetyldehydroaminosäuren erreicht wird (siehe Tabelle 10).

Tabelle 10

| Substratspezifität der N-Acetyldehydroleucin-Acylase | |
|---|---|
| Substrat | rel. Aktivität ( % ) |
| ADL | 100,0 |
| L-Tryptophanamid | 20,4 |
| L-Leucinamid | 6,80 |
| D-Leucinamid | 3,82 |
| L-Methioninamid | 4,69 |
| L-Alaninamid | 0,35 |
| D-Alaninamid | 0,46 |
| 4-Nitrophenylacetat | 1,1 |
| N-Acetyl-glycin | 0 |
| L-Tryptophyl-glycin | 0 |
| L-Leucyl-glycin | 0 |
| L-Alanyl-glycin | 0 |
| Glycyl-L-leucin | 0 |
| L-Alanyl-L-leucin | 0 |
| N-Acetyl-L-alanyl-L-valin | 0 |
| L-Alanyl-L-phenylalaninamid | 0 |
| N-Acetyl-D,L-leucin | 0 |
| N-Acetyl-L-phenylalanin | 0 |
| N-Benzoyl-D,L-leucin | 0 |
| N-Methoxycarbonyl-D,L-leucin | 0 |
| N-Methoxycarbonyl-D,L-valin | 0 |
| N-Methyl-D,L-leucin | 0 |
| N-Methyl-L-glutaminsäure | 0 |
| N-Carbamoyl-L-valin | 0 |
| N-Carbamoyl-L-phenylalanin | 0 |
| Isopropylhydantoin | 0 |
| Dihydrouracil | 0 |

Beispiel 14: Kontinuierliche Herstellung von L-Leucin

N-Acetyldehydroleucin kann enzymatisch zu L-Leucin umgesetzt werden, indem man durch Kopplung der Acylase mit einer L-Leucindehydrogenase das Intermediat 2-Ketoisocaproat stereospezifisch reduktiv aminiert. Die Regenerierung des bei der Dehydrogenierungsreaktion oxidierten Coenzyms erfolgte in Anwesenheit von Formiat durch eine Formiat-Dehydrogenase. Die kontinuierliche Reaktionsführung wurde in einem Enzym-Membranreaktor vorgenommen. Dieser beinhaltete zu Versuchsbeginn:

| | |
|---|---|
| 6,63 U/ml | Acylase (Präparat mit 75 U/ml aus der Aussalzchromatographie) |
| 11,2 U/ml | L-Leucindehydrogenase (Bacillus cereus) |
| 8,4 U/ml | Formiatdehydrogenase (Candida boidinii) |
| 0,6 mM | PEG 20000-NADH (hergestellt gemäß DE-PS 2 841 414) |
| 900 mM | Ammoniumformiat (pH 9) |

Der auf 25° C temperierte Reaktorinhalt (10 ml) wurde mittels einer Schlauchpumpe im Kreislauf über eine Ultrafiltrationsmembran (Typ YM5, Firma Amicon, Ausschlußgrenze 5000 Dalton) gepumpt. Hierbei

können die niedermolekularen Substanzen kontinuierlich entfernt werden, während die Enzyme und das molekulargewichtsvergrößerte Coenzym in der Reaktionslösung zurückgehalten werden. Das Volumen der ultrafiltrierten Produktlösung, etwa 9 ml/Stunde, wurde kontinuierlich durch Substratlösung ersetzt. Die mittlere Verweilzeit betrug demnach 1,1 Stunden. Die Substratlösung beinhaltete in den ersten 53 Betriebsstunden 50 mM N-Acetyldehydroleucin und in den nachfolgenden 57 Stunden 75 mM, jeweils gelöst in 900 mM Ammoniumformiat mit pH 9. Die Produktlösung wurde in Fraktionen gesammelt und deren L-Leucin-Konzentration mittels eines enzymatischen Tests mit der L-Leucindehydrogenase nach der Endpunktmethode bestimmt. Die Testansätze enthielten:

| | |
|---|---|
| 10 % (v/v) | Probe oder Standard mit maximal 2 mM L-Leucin |
| 3,4 mM | $NAD^+$ |
| 4,8 U/ml | L-Leucindehydrogenase |
| 80 mM | Glycinpuffer, pH 10,7 |

Vor dem Reaktionsstart durch Enzymzugabe sowie nach 90 Minuten Inkubation bei Raumtemperatur wurde die Extinktion (340 nm) in den Ansätzen gemessen und aus der Differenz anhand einer Eichkurve die L-Leucinkonzentration bestimmt.

Tabelle 11 zeigt, daß es möglich ist, kontinuierlich mit hoher Ausbeute N-Acetyldehydroleucin zu L-Leucin umzusetzen.

Tabelle 11

| Kontinuierliche Herstellung von L-Leucin aus N -Acetyldehydroleucin | | | |
|---|---|---|---|
| Zeitintervall (h) | N-Acetyldehydroleucin (mM) | Ausbeute an L-Leucin*) ( % ) | Umsatz*) ( % ) |
| 0 - 53 | 50 | 90 | 91 |
| 53 - 110 | 75 | 84 | 86 |

*) zeitliche Mittelwerte

Beispiel 15: Herstellung von L-Tryptophylglycin aus L-Tryptophanamid und Glycin

a) pH-Optimum der L-Tryptophylglycin-Synthese

Unter Einwirkung der ADL-Acylase ist es möglich, L-Tryptophylglycin (L-Trp-Gly) aus L-Tryptophanamid (L-Trp-$NH_2$) und Glycin herzustellen. Als Nebenprodukte entstehen L-Tryptophan (L-Trp) und Ammoniak. In Ansätzen bestehend aus

| | |
|---|---|
| 50 mM | L-Trp-$NH_2$ |
| 200 mM | Glycin |
| 2,6 U/ml | ADL-Acylase |
| 100 mM | Puffer bei pH 7 - 9 |

wurden durch Zugabe von Natronlauge bzw. Phosphorsäure verschiedene pH-Werte zwischen 7 und 11 eingestellt. Als Puffersubstanzen dienten bei pH 7 Kaliumphosphat, bei pH 8 und 9 Tris/Phosphorsäure. Nach 18 Stunden Inkubation bei 25° C wurde die Zusammensetzung der Proben im Vergleich mit Standardlösungen dünnschichtchromatographisch untersucht. Als Laufmittel dienten Eisessig/Butanol/Wasser im Volumenverhältnis 2/8/2 sowie Methylethylketon/Pyridin/Wasser/Eisessig im

Verhältnis 70/15/15/2. Die Detektion erfolgte beim erstgenannten Laufmittel über die UV-Fluoreszenzlöschung bei 254 nm sowie mit dem Ninhydrin-Sprühreagenz, bei letzterem Laufmittel nur mit der Ninhydrinmethode. Die Konzentrationen an L-Trp und L-Trp-Gly wurden anhand von Standards auf dem Aminosäureanalysator bestimmt.

Wie aus Tabelle 12 hervorgeht, liegt das Optimum der Synthesereaktion bei pH 10. Bei diesem pH-Wert werden 94% des eingesetzten L-Trp-$NH_2$ gespalten und hiervon 64% zum Dipeptid umgesetzt (64% Selektivität).

Tabelle 12

| Einfluß des pH-Wertes auf die Synthese von L-Tryptophylglycin | | | |
|---|---|---|---|
| pH-Wert | Dipeptid Ausbeute ( % ) | Selektivität ( % ) | Umsatz ( % ) |
| 7 | 10 | 10 | 100 |
| 8 | 25 | 25 | 100 |
| 9 | 44 | 45 | 98 |
| 9,5 | 53 | 57 | 92 |
| 10 | 60 | 64 | 94 |
| 10,5 | 53 | 63 | 84 |
| 11 | 45 | 62 | 73 |

b) Einfluß der L-Trp-$NH_2$-Konzentration auf die L-Trp-Gly-Synthese

In Ansätzen zur Synthese von L-Trp-Gly aus L-Trp-$NH_2$ und Glycin in Gegenwart der ADL-Acylase (über Aussalzchromatographie gereinigt) wurde die L-Trp-$NH_2$-Konzentration von 24,4 bis 400 mM verändert. Glycin wurde in 400 - 488 molarem Überschuß zum L-Trp-$NH_2$ eingesetzt und die Acylasekonzentration proportional zu der des L-Trp-$NH_2$ erhöht (3,6 U/ml pro 50 mM L-Trp-$NH_2$). Inkubation und Auswertung erfolgten wie unter a) beschrieben.

Im Bereich von 24 bis 182 mM reagieren über 50 % des L-Trp-$NH_2$ zum Dipeptid ab (siehe Tabelle 13).

Tabelle 13

| Synthese von L-Trp-Gly in Abhängigkeit von der L-Trp-$NH_2$-Konzentration | | | |
|---|---|---|---|
| L-Trp-$NH_2$ (mM) | Ausbeute ( % ) | Selektivität ( % ) | Umsatz ( % ) |
| 24,4 | 51 | 72 | 71 |
| 47,6 | 59 | 66 | 89 |
| 95,2 | 59 | 59 | 100 |
| 140 | 55 | 56 | 97 |
| 182 | 51 | 54 | 94 |
| 298 | 36 | 56 | 63 |
| 400 | 12 | 53 | 22 |

c) Beeinflussung der L-Trp-Gly-Synthese durch polare Substanzen im Reaktionsgemisch

Ansätze zur L-Trp-Gly-Synthese aus L-Trp-$NH_2$ (50 mM) und Glycin (500 mM, pH 10) in Gegenwart der ADL-Acylase wurden mit verschiedenen ionischen und ungeladenen polaren Verbindungen in hoher Konzentration versetzt und 19 Stunden bei 25° C inkubiert. Die Acylase wurde teilgereinigt durch Aussalzchromatographie zu 2,6 U/ml und als reines Enzym nach Chromatographie an Mono-Q zu 1,5 U/ml eingesetzt. Danach wurde, wie unter a) beschrieben, der Gehalt an Tryptophanverbindungen in den Reaktionsgemischen bestimmt.

Tabelle 14 zeigt, daß bei 50 % (w/v) Glycerin eine um 12 %-Punkte höhere Selektivität erreicht wird bei gleicher Ausbeute wie im Kontrollansatz.

Tabelle 14

| Einfluß ionischer und ungeladener polarer Substanzen auf die L-Trp-Gly-Synthese | | | | |
|---|---|---|---|---|
| Substanz | Konzentration (% w/v) | Selektivität ( % ) | Ausbeute ( % ) | Umsatz ( % ) |
| teilgereinigte Acylase: | | | | |
| Kontrolle | - | 67 | 64 | 96 |
| Kaliumphosphat | 5,0 | 59 | 59 | 100 |
| Kaliumphosphat | 10,0 | 48 | 48 | 100 |
| Ammoniumsulfat | 12,5[a)] | 65 | 65 | 100 |
| Ammoniumsulfat | 25,0[a)] | 63 | 68 | 89 |
| Ethanol | 10,0[b)] | 66 | 66 | 100 |
| Ethanol | 20,0[b)] | 72 | 64 | 89 |
| Glycerin | 25,0 | 71 | 71 | 100 |
| Glycerin | 50,0 | 79 | 65 | 83 |
| reine Acylase: | | | | |
| Kontrolle | - | 73 | 62 | 85 |
| Glycerin | 25 | 78 | 56 | 72 |
| Glycerin | 50 | 83 | 54 | 66 |

a) % Sättigung
b) % v/v

Beispiel 16: Herstellung von L-Trp-Dipeptiden aus L-Trp-$NH_2$ und D- bzw. L-Aminosäuren

Es wurde untersucht, ob auch D- und L-Aminosäuren anstelle des Glycins als Aminokomponenten für die Dipeptidsynthese aus L-Trp-$NH_2$ in Gegenwart der ADL-Acylase dienen können. Die D- und L-Aminosäuren wurden nahe ihrer Löslichkeitsgrenze bzw. zu maximal 0,5 M in Tests mit folgender Zusammensetzung eingesetzt:

| | |
|---|---|
| 100 - 500 mM | D- oder L- Aminosäure |
| 50 mM | L-Trp-$NH_2$ |
| 2,6 U/ml | Acylase (gereinigt mittels Aussalzchromatographie) |
| pH-Wert 10 | |

Die Ansätze wurden 19 Stunden bei 25° C inkubiert und anschließend mit dem Aminosäureanalysator und über Dünnschichtchromatographie auf ihren Gehalt an Ninhydrin-positiven Verbindungen untersucht (Laufmittel Methylethylketon/Pyridin/Wasser/Eisessig im Verhältnis 70/15/15/2). Aminosäuren sowie die Dipeptide L-Trp-Gly, L-Trp-D,L-Alanin und L-Trp-D,L-Phenylalanin wurden im Vergleich mit Standardlösungen identifiziert und quantifiziert. Zusätzliche Peaks in den übrigen Analysatorchromatogrammen wurden in Analogie auch als Dipeptide interpretiert und deren Konzentration anhand der mittleren spezifischen Peakfläche o.g. Standardlösungen (+ 10 % Standardabweichung) abgeschätzt.

Wie aus Tabelle 15 hervorgeht, werden mit den Aminosäuren D-Methionin und D-Cystein etwa 40 % bzw. 15 % des L-Trp-$NH_2$ zum entsprechenden Dipeptid umgesetzt.

Tabelle 15

| D- und L-Aminosäuren als Aminokomponenten zur Synthese von Tryptophyldipeptiden | | | | | |
|---|---|---|---|---|---|
| Aminokomponente | Konz. (mM) | Ausbeute ( % ) | Umsatz ( % ) | Selektivität ( % ) | Berechnungsart |
| Glycin | 500 | 66 | 100 | 66 | eig.St. |
| D-Methionin | 300 | 39 | 99 | 40 | mittl.St. |
| D-Leucin | 150 | 23 | 100 | 23 | mittl.St. |
| L-Methionin | 300 | 22 | 95 | 22 | mittl.St. |
| D-Alanin | 500 | 22 | 87 | 26 | eig.St. |
| D-Cystein | 500 | 15 | 85 | 17 | mittl.St. |
| L-Alanin | 500 | 12 | 99 | 12 | eig.St. |
| D-Valin | 500 | 12 | 91 | 13 | mittl.St. |
| D-Phenylalanin | 150 | 11 | 59 | 18 | eig.St. |
| D-Serin | 500 | 10 | 100 | 10 | mittl.St. |
| L-Cystein | 500 | 3 | 94 | 3 | mittl.St. |
| L-Leucin | 150 | 0 | 100 | 0 | - |
| L-Valin | 500 | 0 | 93 | 0 | - |
| L-Phenylalanin | 150 | 0 | 100 | 0 | - |
| D-Tryptophan | 100 | 0 | 100 | 0 | - |
| L-Tryptophan | 100 | 0 | 100 | 0 | - |
| D-Histidin | 150 | 0 | 83 | 0 | - |
| L-Histidin | 150 | 0 | 91 | 0 | - |
| D-Prolin | 500 | 0 | 100 | 0 | - |
| L-Prolin | 500 | 0 | 100 | 0 | - |
| L-Lysin | 500 | 0 | 100 | 0 | - |
| L-Arginin | 150 | 0 | 100 | 0 | - |
| L-Glutaminsäure | 500 | 0 | 77 | 0 | - |
| eig. St.: eigener Eichstandard;<br>mittl. St.: mittlerer Standard aus Eichungen mit 4 Tryptophyldipeptiden (siehe oben); | | | | | |

Beispiel 17: Enantioselektivität der Tryptophyldipeptid-Synthese mit der N-Acetyldehydroleucin-Acylase

Die Stereospezifität der ADL-Acylase bei der Synthese von Dipeptiden wurde bezüglich der Carboxylkomponente Trp-$NH_2$ und der Aminokomponente Methionin untersucht. Die im folgenden beschriebenen Ansätze wurden 25 Stunden bei 25° C inkubiert.

| | |
|---|---|
| 50 mM | L- oder D,L-Trp-$NH_2$ |
| 300 mM | D- oder L-Methionin oder 500 mM Glycin |
| 2,6 U/ml | Acylase |
| pH Wert 10 | |

Die Aminosäure- und Dipeptidkonzentrationen wurden, wie unter Beispiel 16 beschrieben, ermittelt.

Als Carboxylkomponente bevorzugt die Acylase das L-Enantiomer des Trp-$NH_2$ und als Aminokompo-

nente die D-Form des Methionins (siehe Tabelle 16).

Tabelle 16

| Dipeptidsynthese in Abhängigkeit von der Stereokonfiguration der Ausgangsverbindungen | | | | | | |
|---|---|---|---|---|---|---|
| $Trp-NH_2$-Stereokonfig. | Aminokomponente/Konz. (mM) | Dipeptid | $t_r$ (Min) | Ausbeute (%) | Umsatz (%) | Selektivität (%) |
| L | Gly / 500 | L-Trp-Gly | 49,77 | 65 | 100 | 65 |
| D,L | Gly / 500 | D,L-Trp-Gly | 49,79 | 60 | 100 | 60 |
| L | L-Met/ 300 | L-Trp-L-Met | 49,31 | 25 | 97 | 26 |
| L | D-Met/ 300 | L-Trp-D-Met | 49,92 | 50 | 100 | 50 |
| D,L | L-Met/ 300 | L-Trp-L-Met | 49,29 | 15 | 84 | 19 |
| D,L | D-Met/ 300 | L-Trp-D-Met | 49,87 | 44 | 93*) | 48*) |
| | | D-Trp-D-Met | 49,33 | 39 | 93*) | 42*) |
| | | gesamt | | 42 | 93 | 45 |

*) unter Annahme gleichen Umsatzes beider Antipoden des $Trp-NH_2$;
Gly: Glycin;
Met: Methionin;
$t_r$: Retentionszeit

## Ansprüche

1. Mikrobiologisch hergestellte N-Acetyl-2,3-didehydroleucin-Acylase, gekennzeichnet durch die folgenden Eigenschaften:

1) Reaktivität:
sie spaltet die Acetylgruppe von N-Acetyl-2,3-didehydroleucin ab, wobei Essigsäure und nach chemischen Folgereaktionen 2-Keto-4-methyl-pentansäure und Ammoniak als Endprodukte entstehen;

2) Substratspezifität:
sie hydrolysiert sowohl verschiedene N-Acetyl-2,3-didehydroaminosäuren, wie N-Acetyl-2,3-didehydrovalin, -isoleucin, 2-Acetylaminozimtsäure und 2-Acetylaminoacrylsäure als auch Aminosäureamide, wie D- und L-Tryptophanamid, D-und L-Leucinamid und L-Methioninamid, nicht aber 2,3-gesättigte N-Acetylaminocarbonsäuren wie N-Acetylleucin oder -valin;

3) Optimaler pH-Wert:
der optimale pH-Wert ist 9,3 ± 1;

4) pH-Stabilität:
sie zeigt gute Stabilität im pH-Bereich zwischen 9 und 10;

5) Optimale Temperatur:
die optimale Temperatur beträgt 55° C bei einem pH-Wert von 9;

6) Temperaturbeständigkeit:
bei 50° C sind nach 30 Minuten Inkubation noch 90% Restaktivität nachweisbar;

7) Einflüße von Inhibitoren und Aktivatoren:
inhibierend wirken Hemmstoffe von Serinproteasen, insbesondere Phenylmethansulfonylfluorid (0,001 mM), Glycin beschleunigt die Substratspaltung konzentrationsabhängig;

8) Molekulargewicht:
Das Molekulargewicht beträgt etwa 60000;

9) Untereinheiten:
das Molekül besteht aus nur einer Einheit;

10) $K_M$-Wert:
der $K_M$-Wert für das Substrat N-Acetyl-2,3-didehydroleucin beträgt 4,5 mM (30° C, 0,1 M Glycinpuffer, pH 9).

2. Verfahren zur Gewinnung der N-Acetyl-2,3-didehydroleucin-Acylase gemäß Anspruch 1,
dadurch gekennzeichnet,
daß man Zoogloea ramigera DSM 4306 in einem wässrigen Nährmedium, das eine Quelle für Kohlenstoff

und Stickstoff und Mineralsalze enthält, bei einem Ausgangs-pH-Wert zwischen 7,5 und 9 aerob bei 25 bis 38° C anzieht, dann N-Acetyl-2,3-didehydroleucin als Induktor der Nährlösung zusetzt und bei pH-Wert 6,5 weiterhin aerob bei 28 bis 38° C kultiviert, die Zellmasse abtrennt und das Enzym aus den Zellen isoliert.

3. Verwendung der N-Acetyl-2,3-didehydroleucin-Acylase gemäß Anspruch 1 als Bestandteil eines gekoppelten Enzymsystems für die enzymatische Umsetzung, die über die Zwischenstufen 2-Imino-4-methyl-pentansäure bzw. 2-Keto-4-methyl-pentansäure zu L-Leucin verläuft.

4. Verwendung der N-Acetyl-2,3-didehydroleucin-Acylase gemäß Anspruch 1 für die enzymatische Umsetzung von D-oder L-Tryptophanamid und Glycin zum Dipeptid D-oder L-Tryptophylglycin, von D-oder L-Tryptophanamid und D-Methionin zum D- oder L-Tryptophyl-D-Methionin sowie von L-Tryptophanamid und D-Cystein zum Dipeptid L-Tryptophyl-D-Cystein.

Europäisches Patentamt

# EUROPÄISCHER RECHERCHENBERICHT

Nummer der Anmeldung

EP 90 10 1390

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 252 216 (DEGUSSA AG)<br>* Ansprüche *<br>--- | 1 | C 12 N 9/80 |
| A | EP-A-0 201 039 (MICROBIAL CHEMISTRY RESEARCH FOUNDATION)<br>* Ansprüche *<br>----- | 1 | |
| | | | **RECHERCHIERTE SACHGEBIETE (Int. Cl.5)** |
| | | | C 12 N |

Der vorliegende Recherchenbericht wurde für alle Patentansprüche erstellt

| Recherchenort | Abschlußdatum der Recherche | Prüfer |
|---|---|---|
| DEN HAAG | 04-05-1990 | HUBER A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)